# EUROPEAN PATENT APPLICATION

(11) **EP 2 233 124 A1**
(43) Date of publication of application: **29.09.2010**
(21) Application number: 10154479.9
(22) Date of filing: 24.02.2010
(51) Int. Cl.: A61K 8/02, A61K 8/31, A61K 8/58, A61K 8/67

(54) **Textile support impregnated with a cleansing/moisturizing composition**

(30) Priority: 27.03.2009 IT MI20090480
(71) Applicant: BIO.LO.GA. S.r.l., 31015 Conegliano (TV) (IT)
(72) Inventor: Panin, Giorgio, 45100, Rovigo (IT)
(74) Representative: Ferreccio, Rinaldo

(57) **Abstract**

A textile support is described, such as for example a tissue or a gauze, impregnated with a cleansing/maisturizing composition comprising a volatile oil selected from among volatile silicones, hydrogenated polyisobutene, hydrogenated polydecene and mixtures of hydrogenated polyisobutene and/or hydrogenated polydecene with hydrogenated polyolefines, and at least 10% of a compound selected from among vitamin E and esters thereof, more particularly vitamin E acetate, by weight of the total weight of the composition,

## Description

### Field of application

The present invention relates to the sector of the cosmetics industry.

More particularly the invention relates to a textile support, such as for example a tissue or a gauze, impregnated with a liquid composition with cleansing and moisturizing properties.

### Prior art

Use of tissues impregnated with cleansing and/or moisturizing or emollient solutions or emulsions has now become daily; good examples are the cleansing tissues used for the personal hygiene of babies and children in general and the cleansing/ deodorant type used also by adults, above all where there is no water (e.g. when traveling or during excursions, etc.). The tissues in question can be packaged individually in sachets or dispensed by special hermetically sealed packaging which contains a plurality thereof.

Examples of prior patent documents relating to tissues impregnated with cleansing and/or moisturizing or emollient compositions are given herein below.

US 6 187 695 describes a fabric with a cellulose base (for example a tissue for the face) comprising a refreshing composition applied to the external surface of the fabric. One of the refreshing compositions given as an example comprises dimethicone, purified mineral oil, cetyl acetate and acetylated lanolin alcohol, tridecyl pentanoate, tocopherol and cerasin wax.

US 2005/0002994 relates to cosmetic or dermatological tissues comprising a water-insoluble nonwoven fabric impregnated and humidified with a w/o emulsion which may comprise vitamin E or a derivative thereof and up to 25% of a silicone oil.

EP 1 491 186 relates to an article for cleansing the skin or hair, made from a fabric composed of fibers of cotton and fibers of viscose and polyester, wherein the content of cotton fibers increases when moving towards the surface, and containing a cosmetic composition chosen from the group comprising oils, silicone oils, emulsions, dispersions, alcoholic solutions and aqueous solutions. The silicone oil can be for example cyclomethicone or dimethicone and the cosmetic composition can comprise additives, including vitamin E. The example is given, among others, (example 16), of an oily formula for the impregnation of the abovementioned fabrics, based on cyclomethicone and containing 2% of vitamin E, in addition to 3.5% of other ingredients.

US 2005/0276825 describes tissues impregnated with oil, which must be used after a bath. Among the oils used for impregnating the tissues mention is made of olive oil, mineral oil, cocoa butter and vitamin E.

It is also known from EP 1 342 473 B1 that the esters of vitamin E, applied as such, have a marked moisturizing effect on the skin. However the impregnation of tissues or similar textile supports with just vitamin E or esters thereof does not allow a skin cleansing action to be obtained.

WO 2008/022186 describes an antimicrobial sanitizing formulation with skin protection properties; in one example a formulation is disclosed containing 8% vitamin E, 8% of EOP-122, which is 39% active in vitamin E acetate, and dimethicone, a non-volatile silicone.

WO 99/26572 describes an antioxidant composition for topical/transdermal prevention and treatment of wrinkles; example VII of this document relates to a matrix patch comprising volatile cyclomethicone and up to 10% vitamin E. No reference is made to any potential textile nature of the patch.

EP 0 998 943 discloses a vitamin E acetate-based hydrophobic gel formulation for topical application. Such a formulation is aimed at protecting and moisturizing the skin and can also be used as an excipient of pharmaceutically active substances. It contains 20 to 70% of vitamin E acetate, 20 to 70% of a volatile silicone and 7 to 13% of hydrogenated castor oil. The latter component provides for the formation of the three-dimensional structure of the hydrophobic gel. This hydrophobic gel is not suitable for use as a cleansing formulation and even less for impregnating tissues or similar textile supports therewith.

### Summary of the invention

The problem at the basis of the present invention has been that of making available tissues or similar textile supports impregnated with a liquid composition which, in addition to cleansing the skin, is likewise able to exert a marked moisturizing, emollient and soothing action.

This problem has been solved, according to the invention, by a textile support impregnated with a cleansing/moisturizing composition comprising a volatile oil selected from the group comprising volatile silicones, hydrogenated polyisobutene, hydrogenated polydecene and mixtures of hydrogenated polyisobutene and/or hydrogenated polydecene with hydrogenated polyolefines, more particularly hydrogenated C₆-C₁₄ polyolefines, and at least 10% of a compound selected from among vitamin E and esters thereof, by weight of the total weight of the composition.

Vitamin E can be used as d-α-tocopherol or as a mixture of two enantiomers d and 1 of α-tocopherol or as a mixture of other tocopherols (β, γ, ε, ζ, η) of vegetal origin or as tocotrienols. The different forms of vitamin E can be of a natural or synthetic origin.

The abovementioned volatile silicones are preferably selected from the group comprising pentamer cyclomethicone (or decamethylcyclopentasiloxane), tetramer cyclomethicone (or octamethylcyclotetrasiloxane), hexamer cyclomethicone, hexamethyldisiloxane and the mixtures thereof. Pentamer cyclomethicone is particularly preferred.

The textile support can be made up of a tissue of paper, woven or nonwoven fabric or a natural fiber gauze, for example cotton or linen, or synthetic, for example viscose, polyesters, polyamides or polyolefines.

The abovementioned composition preferably contains from 10% to 50%, in a more preferred manner from 10 to 25% and advantageously from 15 to 20%, of the abovementioned compound selected from among vitamin E and esters thereof, by weight of the total weight of the composition.

The abovementioned compound preferably consists of vitamin E acetate.

The abovementioned composition preferably does not comprise any other ingredient in addition to the abovementioned compound and the abovementioned volatile oil.

The composition can be applied to the abovementioned textile support with various techniques, including impregnation by immersion of the support in the composition, spraying on one or both surfaces of the textile support by means of spray devices and the simple depositing of the composition onto the support, followed by impregnation through diffusion. The application of the tissues according to the present invention on the skin allows an excellent cleansing action to be obtained thanks to the volatile oil contained therein, followed, once the volatile oil has evaporated, by a marked moisturizing and soothing action due to the compound of vitamin E.

### Detailed description

The present invention will be described in greater detail with reference to some examples of embodiments given purely by way of illustration and not of limitation.

### EXAMPLE 1

Tissues made of synthetic fiber (70% fibers of viscose and 30% fibers of polyester with a thickness between 0.53 and 0.67 mm and a weight of 27-33 g/m²) were made available, which were impregnated, by depositing, with a solution composed, in percentages by weight of the total weight of the solution, of 15% vitamin E acetate and 85% pentamer cyclomethicone, The impregnated tissues were then packaged individually in sachets of aluminum/LLDPE (linear low density polyethylene).

### EXAMPLE 2

The synthetic fiber tissues as per example 1 were impregnated, by depositing, with a solution composed, in percentages by weight of the total weight of the solution, of' 18% vitamin E acetate and 82% of a mixture of hydrogenated polyisobutene, hydrogenated polydecene and hydrogenated C₆-C₁₄ polyolefines (product marketed with the name Dedraflow^{®} 5.1 by the firm Cosmetic Innovations & Technologies, France). The impregnated tissues were then packaged individually in sachets of aluminum/LLDPE.

### COMPARATIVE EXAMPLE

The synthetic fiber tissues as per example 1 were impregnated, by depositing, with a solution composed, in percentages by weight of the total weight of the solution, of 2% vitamin E acetate and 98% pentamer cyclomethicone. The impregnated tissues were then packaged individually in sachets of aluminum/LLDPE.

### TEST

A comparison was made between the impregnated tissues according to example 1 (according to the invention) and those according to the comparative example in relation to the moisturizing effect on the skin. The moisturizing effect was valuated by means of skin corneometry with the aid of apparatus known as a corneameter, i.e. a particular capacimeter which, via a skin probe, enables a measurement of the variations in the skin capacitance, induced by a larger or smaller content of water in the more superficial layers of the epidermis.

For this valuation 15 subjects of both sexes (7 of male gender and 8 of female gender) were required to apply, for a period of seven days, an impregnated tissue according to example 1 on the medial surface of the left forearm every 12 hours with a light massage and to apply, by the same methods, an impregnated tissue according to the comparative example on the medial surface of the right forearm. During this period the forearm was only washed every 24 hours.

The subjects were examined on the day of the first application and on the seventh day, in both cases taking a basal measurement, just before the application, and a subsequent measurement 6 hours after the application, obtaining the results reported in the following table.

| | Increase in skin hydration (in corneometric units) | |
|---|---|---|
| | First day | Seventh day |
| EXAMPLE 1 | 4.25 | 2.88 |
| COMPARATIVE EXAMPLE | 3.31 | 1.63 |

Analysis of the values given in the table shows that the tissues according to the present invention determine an increase in skin hydration which is significantly higher than that obtained with the application of the tissues according to the comparison example.

From the standpoint of skin cleansing the tissues according to example 1 and those according to the comparative example have not instead shown any appreciable difference.

The tissues according to example 2 supplied, in a separate test, similar results in terms of skin hydration, with increases at the first and at the seventh day of 4.17 and 2.90 respectively.

## Claims

1. A textile support impregnated with a cleansing/moisturizing composition comprising a volatile oil selected among the group including volatile silicones, hydrogenated polyisobutene, hydrogenated polydecene and mixtures of hydrogenated polyisobutene and/or hydrogenated polydecene with hydrogenated polyolefines, and at least 10% of a compound selected among vitamin E and esters thereof, by weight of the total weight of the composition.

2. An impregnated textile support according to claim 1, wherein said volatile silicones are selected among the group including pentamer cyclomethicone (or decamethylcyclopentasiloxane), tetramer cyclomethicone (or octamethylcyclotetrasiloxane), hexamer cyclomethicone, hexamethyldisiloxane, and mixtures thereof.

3. An impregnated textile support according to claim 1, wherein said volatile oil is pentamer cyclomethicone.

4. An impregnated textile support according to any one of the preceding claims, wherein said textile support consists of a paper-, fabric- or non-woven fabric- tissue or a gauze of natural or synthetic fiber.

5. An impregnated textile support according to any one of the preceding claims, wherein said cleansing/moisturizing composition contains 10% to 50%, by weight of the total weight of the composition, of said compound selected among vitamin E and esters thereof.

6. An impregnated textile support according to any one of claims 1 to 4, wherein said cleansing/moisturizing composition contains 10% to 25%, preferably 15% to 20%, by weight of the total weight of the composition, of said compound selected among vitamin E and esters thereof.

7. An impregnated textile support according to any one of the preceding claims, wherein said compound is vitamin E acetate.

8. An impregnated textile support according to claim 1 or 4, wherein said cleansing/moisturizing composition consists of' a volatile oil selected among the group including volatile silicones, hydrogenated polyisobutene, hydrogenated polydecene and mixtures of hydrogenated polyisobutene and/or hydrogenated polydecene with hydrogenated polyolefines, and at least 10% of said compound selected among vitamin E and esters thereof, by weight of the total weight of the composition.

9. An impregnated textile support according to claim 8, wherein said volatile oil is cyclomethicone and said compound is vitamin E acetate.

10. An impregnated textile support according to claim 9, wherein said cleansing/moisturizing composition contains 10% to 25%, preferably 15% to 20%, by weight of the total weight of the composition, of vitamin E acetate.
